# EUROPEAN PATENT APPLICATION

(11) **EP 2 022 344 A1**
(43) Date of publication of application: **11.02.2009**
(21) Application number: 07113732.7
(22) Date of filing: 02.08.2007
(51) Int. Cl.: A23L 1/29, A23L 1/30, A23G 1/42, A61K 31/353

(54) **Reduction of fatigue as a result of exercise**

(71) Applicant: Nestec S.A., 1800 Vevey (CH)
(72) Inventor: Chou, Chieh Jason, 1005 Lausanne (CH); Cooper, Karen Anne, 1814 La Tour-de-Peilz (CH); Williamson, Gary, Harrogate, HG1 1BY (GB); Gleeson, Micheal,, Burbage near Hinckley, Leics. LE10 2LF, (GB); Davison, Glen,, Ceredigion SY24 5HF, (GB)
(74) Representative: Chautard, Cécile

(57) **Abstract**

Use of at least 25mg of a polyphenol for the preparation of a product to be consumed before or during exercise for reducing fatigue, muscle soreness, perceived exertion or recovery time, and/or improving performance or cognition performance.

## Description

### Field of the Invention

The present invention relates to the reduction of fatigue or muscular damage and/or the improvement of performance or speed of recovery as a result of exercise, by the consumption of polyphenols, more particularly by the consumption of cocoa polyphenols such as catechin and epicatechin and related procyanidins, for instance by the consumption of dark chocolate, cocoa liquor or cocoa powder.

### Background of the Invention

It is well known that exercising causes oxidative stress damage in the worked muscle through the production of free radicals and other reactive oxygen species (ROS). These then induce protein oxidation and may be implicated in muscle fatigue. Muscle cells can be protected from these ROS by a cooperative system of endogenous defence mechanisms, where antioxidants play a role.

There is limited evidence that dietary antioxidant supplementation can improve human performance and reduce immunodepression (i.e susceptibility to conditions such as respiratory tract infections) that can occur after intense exercise, though most of this research has been limited to vitamin C (VC) and vitamin E.

It is well known that polyphenols have potent antioxidant properties and are present in many plant materials such as cocoa, teas, and fruits such as berries. Dark chocolate is one of the richest sources of antioxidant polyphenols. Cocoa polyphenols, most notably catechin and epicatechin, can exert their antioxidant effects in both lipid and water-based environments (amphipathic), meaning they can spare both lipophilic and hydrophilic endogenous antioxidants such as vitamins. There is also evidence that polyphenols can dilate blood vessels, aiding blood flow to the muscles, which may be beneficial for both performance and recovery, and also delay low-density lipoprotein (LDL) oxidation. Kondo *et al.* (1996) observed that following the consumption of 35 g of delipidated cocoa there was a decrease in plasma low density lipoprotein (LDL) susceptibility to oxidation.

Other studies have shown beneficial effects of chronic cocoa or dark chocolate consumption, in healthy participants, (usually consumption of about 100g of dark chocolate per day for 2 weeks or more) on resting blood pressure and insulin sensitivity (Grassi *et al.,* 2005) or on cholesterol profile (increased high density lipoprotein (HDL) concentration and decreased susceptibility of LDL to *ex vivo* oxidation; Mursu *et al*., 2004).

US-6297273B1 discloses cocoa extracts and compounds therefrom such as polyphenols preferably polyphenols enriched with procyanidins and uses for them; for instance, as antineoplastic agents, antioxidants, DNA topoisomerase II enzyme inhibitors, cyclooxygenase and/or lipoxygenase modulators, NO (Nitric Oxide) or NO-synthase modulators, as non-steroidal antiinflammatory agents, apoptosis modulators, platelet aggregation modulators, blood or in vivo glucose modulators, antimicrobials, and inhibitors of oxidative DNA damage.

Example 27 describes the effects of cocoa polyphenols on satiety where by using blood glucose levels as an indicator for the signal events which occur in vivo for the regulation of appetite and satiety, a series of simple experiments were conducted using a healthy male adult volunteer aged 48 to determine whether cocoa polyphenols would modulate glucose levels. The cocoa polyphenols contained no caffeine or theobromine. Fasting blood glucose levels were analyzed on a timed basis after ingestion of 10 fl. oz of Dexicola 75 (caffeine free) Glucose tolerance test beverage with and without 75 mg cocoa polyphenols. Blood glucose levels were measured before ingestion of test beverage, and after ingestion of the test beverage at the following timed intervals: 15, 30, 45, 60, 75, 90, 120 and 180 minutes. A control test solution containing 75 mg cocoa polyphenols dissolved in 10 fl. oz. distilled water (no glucose) was also performed.

Although FIG. 32 shows there is a substantial increase in blood sugar levels obtained very soon after ingestion of a test mixture containing cocoa polyphenols and sugar, it is clear that after 180 minutes the blood glucose level is lower with the glucose tolerance test beverage containing polyphenols and sugar than the two control groups.

However, US-6297273B1 used only small amounts of polyphenols (75mg) in the tests and makes no mention of the tests being carried out after exercise or under stress conditions.

Polyphenols have also been shown to interfere with glucose transport via GLUT 1 and GLUT 4 transporters (Strobel *et al*., 2005) and it has been demonstrated that polyphenols (especially epigallocatechin gallate and epicatechin gallate) enhance insulin activity *in vitro* in the insulin-potentiating epididymal fat-cell assay with rat adipocytes (Anderson & Polansky, 2002). There is limited information however, on the effects of polyphenols on hormone, cytokine and immune responses to exercise and there are limited studies investigating the potential of cocoa containing foods to modulate exercise-induced oxidative stress.

Adequate carbohydrate availability is important for exercise, with plasma glucose being an important energy source. If there are inadequate stores of carbohydrate in the body, then fatigue tends to occur sooner. Consumption of a carbohydrate-rich chocolate bar in the past has shown to increase blood glucose 15 minutes prior to exercise, and to maintain significantly higher blood glucose throughout exercise and post-exercise than the placebo of a carbohydrate-free diet drink (Biomedical and Environmental Sciences 9, 247-255 (1996), Chen et al.). The authors attributed this effect to the carbohydrate content of the chocolate bar (56g bar with 36g carbohydrate and 281 kcal) which is most likely true. However, as the control did not contain any carbohydrate, it is only possible to attribute the effect to carbohydrate, rather than extending it to any innate characteristic of cocoa or chocolate. It is also likely that this chocolate bar contained very little cocoa and there is no suggestion by the authors that the effects are due to the cocoa. The effect is most likely due to the sugar content.

"Clinical & Developmental Immunology, March 2005; 12 (1): 11-17; Cesar G.Fraga et al", discloses that the regular consumption (over 14 days) of a 105g milk chocolate bar containing 168 mg flavanols (of which 39 mg is epicatechin and catechin) can improve oxidant stress and cardiovascular health in fit young soccer players. However, the milk chocolate used did not contain a large amount of flavanols (polyphenols) and blood glucose levels were found to be lower after 14 days consumption of the chocolate compared with baseline values. There was no investigation into the effect of cocoa polyphenols on the control of blood glucose after prolonged exercise with an implication of faster recovery.

There is currently no available literature on the effects of acute dark chocolate consumption on control of blood glucose or oxidative stress following prolonged (> 2 h duration) exercise. None of the above cited documents teaches or suggests that the consumption of polyphenols by people taking exercise can reduce fatigue and muscular damage and improve performance and speed of recovery, nor that the said consumption can inhibit a reduction in post-exercise blood glucose independently of any carbohydrate and fat intake.

Dark chocolate also contains caffeine, which is ergogenic, mobilising fatty acids from stores (through activation of lipolysis) and sparing glycogen, while also stimulating the central nervous system and increasing muscle excitability.

Most of the body's cells rely on glucose as an energy source and blood glucose is an important energy source for exercise. Glucose levels in the blood are very stringently controlled within a range or 70-140 mg/dL (3.9 to 7.8mmol/litre) primarily by hormones such as insulin and glucagon. Immediately after a meal, blood glucose levels rise and exceed the body's immediate energy requirements. The body stores the excess glucose as glycogen in the liver and muscles. A decline in blood glucose is often associated with fatigue and it is known that blood glucose levels fall during and after exercise. Therefore it is accepted that an adequate carbohydrate availability is important for exercise and essential for sustained performance of strenuous prolonged exercise.

However, although the consumption of carbohydrates before exercise to increase the blood glucose level may reduce fatigue and muscular damage, improve performance and aid recovery to some extent, it cannot prevent fatigue and muscular damage taking place while even better performance and speed of recovery are highly desirable.

### Summary of the Present Invention

It has now been found that the consumption of at least 25mg of a polyphenol before or during prolonged or strenuous exercise can reduce fatigue and perceived exertion, improve performance and lead to a faster recovery as well as improving cognition performance during later and post-exercise. It can also can inhibit a reduction in post-exercise blood glucose independently of any carbohydrate and fat intake.

Accordingly, the present invention relates to the use of at least 25mg of a polyphenol for the preparation of a product to be consumed before or during exercise for reducing fatigue, muscle soreness, perceived exertion or recovery time, and/or improving performance or cognition performance.

The reduction in fatigue and perceived exertion may be apparent both during and post-exercise. The improvement in performance may be particularly apparent during the latter stages of the exercise. The reduction in fatigue and muscle soreness may be apparent post-exercise. The improvement in cognition performance may be apparent during later and post-exercise.

The present invention also relates to the use of at least 25mg of a polyphenol for the preparation of a product to be consumed before or during exercise for inhibiting a reduction in post-exercise blood glucose.

### Detailed Description of the Invention

The benefits of the product of the present invention are most apparent when the exercise is prolonged and/or strenuous. By "prolonged" is meant more than 30 minutes, usually more than 1 hour and preferably more than 2 hours. By "strenuous" is meant reaching a heart rate of above 100 to 150 beats per minute or higher depending on the fitness or the age of the participant. The benefits of the composition of the present invention may appear earlier in the duration of the exercise when the exercise is more severe.

The product may be consumed, preferably orally, before the commencement of or during a bout of exercise. Although the product may be consumed several hours before the commencement of the exercise, e.g. up to 8 hours before the exercise, it may be more advantageously consumed at a time closer to the commencement of the exercise, for example less than 30 minutes before, preferably less than 1 hour before, and more preferably from 1.0 to 2.5 hours before the exercise.

The polyphenol may be a polyphenol present in a variety of plant materials such as teas, berries, etc. but the polyphenol used is preferably a polyphenol present in cocoa and may be, for example (but not limited to), catechin, epicatechin, or oligomers of epicatechin such as dimer B2, dimer B5, trimer C or tetramer D. A single polyphenol may be used or mixtures of two or more polyphenols may be used.

When a single polyphenol is used, a smaller amount of a single polyphenol may be as effective as a larger amount of a mixture of polyphenols.

For example, when epicatechin is used singly, the amount of epicatechin in the product may be at least 25mg, conveniently from 30 mg to 200 mg, preferably from 40 mg to 150 mg and more preferably from 50 mg to 100 mg.

When a mixture of polyphenols is used, the amount of total polyphenol in the product (as measured by the folin assay in epicatechin equivalents) may be at least 50 mg, for instance from 100 mg to about 2500 mg, preferably from 200 mg to 2000 mg, more preferably from 500 mg to 1,500 mg, and even more preferably from 750 mg to 1250 mg.

The product containing the polyphenol to be consumed orally may be in solid or liquid form and may be a solution or suspension or powder. For instance, it may consumed as cocoa liquor, cocoa powder, milk chocolate or dark chocolate. It may also be consumed within a foodstuff such as a cereal, a dairy product, e.g. a yoghurt, an energy bar, a gel, or as a drink such as a cocoa or chocolate drink. The product may also be consumed in the form of a dietary supplement such as a capsule, tablet, pill, lozenge, dragee or powder.

For example, depending on the polyphenol content of the cocoa, the amount of dark chocolate consumed before a bout of exercise is conveniently from 20 to 150 grams, preferably from 50 to 125 grams and more preferably from 90 to 110 gram, e.g. a 100 gram bar. The amount of cocoa liquor consumed before a bout of exercise is conveniently from 10 to 90 grams, preferably from 30 to 80 grams and more preferably from 55 to 70 grams. The lower amounts of these products, e.g. 20-50 grams of dark chocolate or 10-30 grams of cocoa liquor might give satisfactory benefits when the polyphenol content of the chocolate is high, e.g. about 1000-1600 mg or more polyphenols per 100 grams of chocolate as measured by the folin-ciocalteau method.

Advantageously, up to 1g of caffeine and/or up to 2g of theobromine which are ergogenic substances may be present in the product containing the polyphenols. For example, the amount of caffeine may be from 0.1 to 0.5 gram and the amount of theobromine may be from 0.1 to 1 grams. Caffeine and theobromine are normal ingredients of cocoa.

The present invention also provides a method of inhibiting a reduction in post-exercise blood glucose by consuming a product comprising at least 25mg of one or more polyphenols before or during a bout of exercise. The product is conveniently consumed less than 30 minutes before the bout of exercise, preferably preferably less than 1 hour before, and more preferably from 1.5 to 2.5 hours before the bout of exercise.

Although not confirmed we believe that the mechanism by which this higher blood glucose is reached is possibly by gluconeogenesis (the making of new glucose for use by the body) or glycogenolysis (breakdown of stored glycogen to release glucose for use by the body) or decreasing overall body turnover of glucose.

The consumption of a product comprising at least 25mg of one or more polyphenols before or during a bout of exercise may also increase the post-exercise blood polyphenol content and inhibit a reduction in post-exercise blood insulin content.

The consumption of the product comprising at least 25mg of one or more polyphenols may also provide one or more of the following additional benefits :
increases fat mobilisation post-exercise :
increases beta-oxidation post-exercise :
increases gluconeogenesis post- and during exercise :
increases glycogenolysis post- and during exercise :
decreases overall body turnover of glucose post- and during exercise :
decreases post-exercise immunodepression :
reduces post-exercise changes of some hormones, e.g. insulin :
reduces post-exercise plasma free F2-isoprostane concentration :
inhibits the reduction of post-exercise plasma insulin (insulinaemia).

A second embodiment of the present invention is related to persons who take regular exercise, e.g. on a daily basis. The benefits may build up over a period of time by the consumption of a daily dose of from 5 to 200 mg of one or more polyphenols. The benefits may be felt within a few days and may last indefinitely if consumption is continuous.

Accordingly, the second embodiment of the present invention relates to the use of from 5 mg to 200 mg of one or more polyphenols for the preparation of a product to be consumed on a daily basis for reducing fatigue, muscle soreness, perceived exhaustion or recovery time, and/or improving performance or cognition performance.

The second embodiment of the present invention also relates to the use of from 5 mg to 200 mg of one or more polyphenols for the preparation of a product to be consumed on a daily basis for inhibiting a reduction in post-exercise blood glucose in a person taking regular exercise.

The benefits of the use of the product of the second embodiment of the present invention are also most apparent when the exercise is prolonged and/or strenuous. By "prolonged" is meant more than 30 minutes, usually more than 1 hour, and preferably more than 2 hours. By "strenuous" is meant reaching a heart rate of above 100- 150 beats per minute or more depending on the fitness or age of the participant.

The second embodiment of the present invention also provides a method of reducing fatigue, muscle soreness, perceived exhaustion or recovery time, and/or improving performance or cognition performance in a person taking regular exercise by consuming daily a product comprising from 5 to 200mg of one or more polyphenols.

The amount of polyphenol consumed on a daily basis may conveniently be from 25 to 150mg and preferably from 50 to 100mg per day.

The amount of epicatechin consumed on a daily basis may be from between 6mg and 80mg and preferably from 10mg to 50mg.
If desired, the daily amount may be taken in one or more portions per day.
When a product containing the higher amounts of the polyphenol or mixture of polyphenols, e.g 100-200mg, is consumed daily it is advisable that items with a similar calorific value already in the diet should be omitted. Dietary advice should be provided with these products to replace similar items already in their diet.

In the second embodiment of the present invention, the product containing the polyphenol or polyphenols to be consumed orally may also be in solid or liquid form and may be a solution or suspension or powder. For instance, it may consumed as cocoa liquor, cocoa powder, milk chocolate or dark chocolate. It may also be consumed within a foodstuff such as a cereal, a dairy product, e.g. a yoghurt, an energy bar, a gel, or as a drink such as a cocoa or chocolate drink. The product may also be consumed in the form of a dietary supplement such as a capsule, tablet, pill, lozenge, dragee or powder.

### EXAMPLE

The following Example further illustrates the present invention.

### Example 1

The test chocolate used (DC) was a 100 gram tablet of dark chocolate (70% cocoa solids) containing cocoa liquor, sugar, cocoa butter, milk fat, lecithin and vanilla having a total fat content of 44%. The control chocolate ((CON) of iso-fat, iso-carbohydrate control was as closely matched as possible in terms of fat and carbohydrate content and total energy. 71 g of the control chocolate (CON) contained all of the same ingredients as the test chocolate (DC) except the cocoa liquor (0%) but contained 41% cocoa butter to obtain a fat content of 62% so that 71 g of the control chocolste contained the same amount of fat as 100 g of DC.

14 fasting, healthy men aged 21-23 and weighing from 70-73kg completed a food record diary for the 48 h period before the first trial (habituation) and were required to follow the same diet during the 48 h prior to each main trial. They were all non-smokers and were required to abstain from alcohol, caffeine and heavy exercise for 48 h prior to each trial and to have a rest day on the day immediately before each trial. It was also stipulated that participants should not take any mineral or vitamin supplement or any other antioxidant supplements during and for the 4 weeks before the study. Participants were provided with a food exclusion list to ensure that they avoided high polyphenol containing foods for the 48 h before each main trial.

The participants were fed in a controlled randomised manner either 100g of dark test chocolate (DC), 71g of iso-fat, iso-carbohydrate control (CON) or nothing (NONE).

After 2 hours, each participant cycled for 2.5 hours at 60% VO₂ max. There was no difference between trials for any of the variables related to physiological demand of the exercise as summarised in Table 1.

**Table 1. Exercise data: averaged over the whole 2.5 h duration of each trial.**

| | DC | CON | NONE |
|---|---|---|---|
| Heart rate (beats.min-1) | 143(4) | 142(4) | 140(4) |
| Rating of Perceived Exertion (RPE) | 12(0) | 12(0) | 12(0) |
| VO2max % | 57(1) | 57(1) | 57(1) |
| RER | 0.94(0.02) | 0.93(0.01) | 0.93(0.02) |

| | | | |
|---|---|---|---|
| Values are mean (SEM). CON (control chocolate trial), DC (dark chocolate trial), NONE (no intervention trial). | | | |

Blood was taken prior to chocolate consumption, 2 hours later just before exercise, just after 2.5 hours exercise and after 1 hour rest. Blood samples were tested for insulin, polyphenols (epicatechin), glucose and plasma free F2-isoprostane. Post-exercise fatigue and perceived exertion were recorded.

The participants exercised in a randomised-counterbalanced manner under 3 different conditions:
a) The NONE trial (no intervention),
b) the DC (dark chocolate) trial, or
c) the CON (control cocoa liquor-free chocolate) trial
They arrived at the laboratory at 08:30 on the morning of the main trials after an overnight fast (>10 h). Participants consumed the chocolate (or nothing in the NONE trial) and 200 ml of water 2 hours prior to beginning exercise. Additionally, 2.5 ml.kg⁻¹ body mass of water was consumed at the onset, every 15 min during, and on completion of the exercise bout.

Venous blood samples were taken as follows:
a) before chocolate consumption (Rest),
b) immediately before beginning exercise (Pre-Ex),
c) immediately after completing the bout (Post-Ex), and
d) after 1 h of recovery (1 h Post-Ex).
On each occasion 25 mL blood was obtained from an antecubital vein using a 21 g butterfly needle and syringe. Blood was then dispensed into three Vacutainer tubes (Becton Dickinson, Oxford, UK): two K₃EDTA tubes and one Heparin tube. Expired gas was collected into Douglas bags (1 min sample), during the 15^{th} and 30^{th} min and every 30 min thereafter during exercise, for analysis of VO₂ and RER (Respiratory Exchange Ratio). Heart rate and rating of perceived exertion (RPE) were recorded every 15 min during exercise.

The order in which each participant undertook each condition (DC, CON or NONE trials) was randomised and counterbalanced using a basic Latin Squares design. There are 6 different possible sequences in which the DC, CON and NONE trials could be undertaken. Participants were assigned randomly into each of the possible sequences resulting in 2 participants in each of the possible sequences. Since there were 14 participants 2 out of the 6 sequences each had an additional participant.

Blood samples in two K₃EDTA vacutainers were used to determine changes in the plasma concentrations of glucose, insulin, polyphenols, plasma free F₂-isoprostane concentration, triglycerides and free fatty acids and heparin tubes were used to determine changes in the plasma concentrations of vitamin C, vitamin E.

To compare the 3 different trials, DC, CON and NONE, a 2-way repeated measures ANOVA (trial x time) was used. Data that were not normally distributed were normalised with log or square root transformation. *Post hoc* analysis was carried out, where appropriate, using paired samples *t* tests with the Holm-Bonferoni correction. When the 2-way ANOVA showed a significant trial × time interaction (indicating different temporal responses depending on trial) then 1-way repeated measures ANOVA was also used to determine the temporal response in each trial independently. The Greenhouse Geisser correction was applied to all ANOVA P values.

### A. PLASMA POLYPHENOL CONCENTRATION

Aliquots of K₃EDTA plasma were analysed to determine the concentration of polyphenols (epicatechin and catechin) using HPLC. Briefly, thawed plasma was centrifuged at 4°C at 14000 rpm for 5 min and 200 µl was mixed with 12 µl of 10% ascorbic acid-40 mM KH₂PO₄-0.1% EDTA, 20 µl of 50 mM potassium phosphate (pH 7.4), 20 µl of 1.0 µg/ml catechin gallate as internal standard, 500 units of β-d-glucuronidase type X-A from *E. coli* (Sigma Chemical Co, St Louise, MO, USA) and 4 units of sulfatase type VIII from abalone entrails (Sigma). The mixture was incubated at 37°C for 45 min. The reaction was stopped by the addition of 2 ml of ethyl acetate followed by vigorous shaking for 20 min and centrifugation at 4°C at 2000 g for 5 min. The supernatant was transferred to a clean tube and the ethyl acetate extraction was repeated. 10 µl of 0.02% ascorbic acid: 0.005% EDTA was added to the pooled supernatant fraction and vortex mixed. The supernatant was then evaporated to dryness with nitrogen for 2 h at room temperature. The samples were reconstituted in 200 µl of methanol:water (1:2 vol), vortexed well, sonicated for 10 min, and centrifuged at 4°C at 14000 rpm for 5 min. 20 µl of the supernatant was injected into the HPLC system which consisted of an ESA Model 582 solvent delivery stystem, an ESA Model 542 autosampler, an ESA 5600 CoulArray electrochemical detector (ESA, Bedford, MA, USA) with CoulArrayWin Software 1.12, an Eppendorf CH-30 Column Heater, a C₁₈ Phonomenex guard column (4.0 mm L x 3.0 mm), and a SUPELCO Ascentis RP-Amide column (15 cm x 4.6 mm i.d., 5 µm particles). Standard flavanol solutions and internal standard (catechin gallate) were prepared in a methanol and water (1:2 vol) solution and stored at -70°C. The column was eluted at 35°C starting at a flow rate of 1 ml/min with 70% buffer A (40 mM ammonia phosphate monobasic, pH adjusted to 3.0 with phosphoric acid) and 30% buffer B (40 mM ammonia phosphate monobasic 50%/L: 50% acetonitrile/L, pH adjusted to 3.0 with phosphoric acid). After 1 min, the gradient was linearly changed with from 70% buffer A and 30% buffer B to 10% buffer A and 90% buffer B (1-10 min), 70% A / 30% B (10-12 min). The eluent was monitored by electrochemical detection with potential settings at -30, 100, 230 and 400 mV. The dominant channel was at 230 mV.

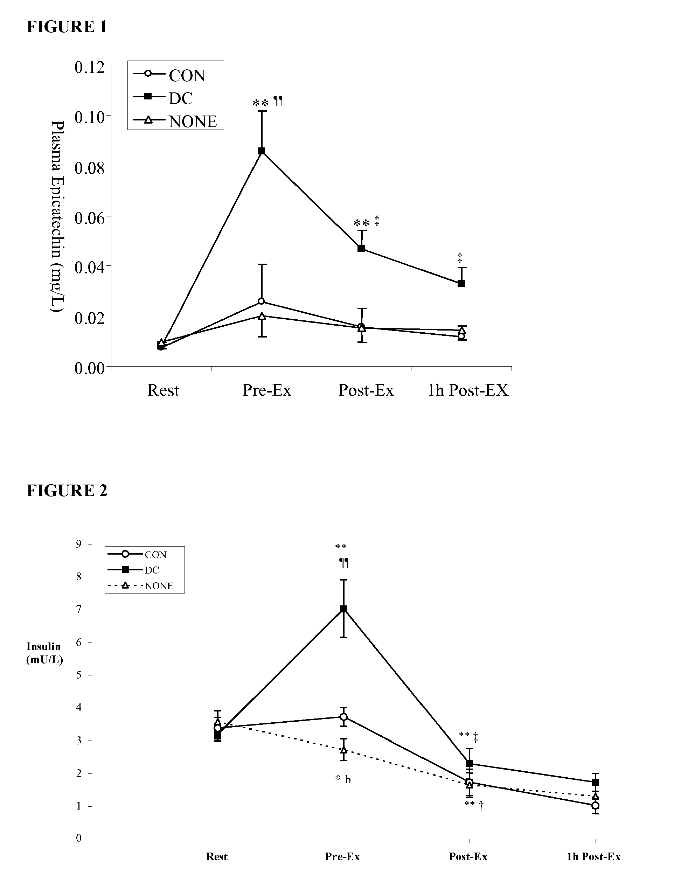

Changes in plasma epicatechin concentrations are shown in Figure 1. Post hoc analysis showed that plasma epicatechin concentration was significantly higher Pre-Ex (i.e. 2 hours postprandial) in the DC trial when compared with the CON trial. Two hours following ingestion of DC the plasma epicatechin concentration was elevated 11-fold. The plasma epicatechin concentration was 6-fold higher than Rest at the end of exercise and by 1 h Post-Ex was about 4-fold higher than Rest. On both the CON and NONE treatment, the plasma epicatechin concentration remained unchanged from Rest throughout the trials. The plasma catechin concentration was not affected by DC or CON ingestion.

### B. PLASMA VITAMINS C AND E CONCENTRATION

Plasma VC concentration was determined in plasma obtained from heparinised blood according to Liu *et al.* (1982) using a specific spectrophotometric ascorbate oxidase (E 1.10.3.3) assay. Plasma trolox equivalent antioxidant capacity (TEAC) was determined in plasma obtained from heparinised blood on an automated analyser (Cobas-Mira Plus, Roche, Basle, Switzerland) using a commercially available kit (Randox, County Antrim, UK) for measuring the capacity of plasma to scavenge the 2,2'-azinobis-(3-ethylbenzothiazoline-6-sulphonic acid) (ABTS) radical cation. Trolox was used as the standard therefore this measure of total antioxidant status (TAS) was expressed in Trolox equivalent antioxidant units (µM). Plasma vitamin E equivalent antioxidant capacity was determined in plasma obtained from heparinised blood using a CL test for measuring the capacity of plasma to scavenge peroxynitrite (ABEL, Knight Scientific, Plymouth, UK).

There was a significant effect of time for plasma Vitamin C concentration. Post hoc analysis showed that plasma vitamin C concentration was higher than Pre-Ex at Post-Ex and 1h Post-Ex (Table 2). There was a significant main trial × time interaction effect and main effect of time for plasma Vitamin E equivalents concentration (Table 2). The plasma Vitamin E equivalents concentration was significantly higher at Post-Ex and 1 h Post-Ex than at Rest and Pre-Ex for all three trials. However, the Vitamin E equivalents concentration was significantly higher at 1 h Post-Ex in the DC and CON trials compared with NONE trial.

### C. PLASMA FREE F₂-ISOPROSTANE CONCENTRATION

For the determination of plasma free F₂-isoprostane, K₃EDTA plasma samples were stored at -80°C in the presence of 0.005% 3,5-Di-tert-4butylhydroxytoluene prior to analysis. Plasma free F₂-isoprostane concentration was determined using an ELISA kit according to the manufacturer's instructions (Cayman Chemical Co., MI, USA).

As shown in Table 2, the plasma free F₂-isoprostane concentration was significantly higher Pre-Ex in the DC compared with the NONE trial but was significantly lower in the DC compared with NONE trial at 1h Post-Ex. 1-way ANOVA on each trial individually showed that for the CON trial F₂-isoprostane concentration was significantly higher than the Pre-Ex levels at Post-Ex but not 1 h Post-Ex and on the NONE trial it was significantly higher than the Pre-Ex levels at Post-Ex and 1 h Post-Ex. A lowering of F2-isoprostanes is considered beneficial.

| **Table2.** Plasma vitamins C & E, and plasma free F₂-isoprostane concentration. | | | | | | |
|---|---|---|---|---|---|---|
| | | Rest | Pre-Ex | Post-Ex | 1 h Post-Ex | Main Effects P values |
| | | | | | | (trial; time; trial x time) |
| Vit C | | | | | | |
| (µM) | DC | 57(4) | 55(4) | 69 (4) | 60 (4) | 0.158; <0.001; 0.453 |
| | CON | 56(4) | 53 (5) | 68 (4) | 58(5) | |
| | NONE | 57(4) | 56 (3) | 74 (6) | 65 (5) | |
| | | | | | | |
| Vit E | | | | | | |
| (µM) | DC | 588(16) | 609 (15) | 746 (22) | 742 (21) | |
| | CON | 608 (14) | 580 (18) | 753 (18) | 758 (21) | 0.353 ; <0.001; 0.001 |
| | NONE | 614 (16) | 606 (13) | 739 (22) | 699 (18) | |
| | | | | | | |
| F₂-isoprostane | | | | | | |
| (pg.ml⁻¹) | DC | 14.4(0.4) | 14.8(0.6) | 16.1(0.9) | 14.3 (0.5) | 0.252 ; <0.001; 0.014 |
| | CON | 14.1(0.5) | 13.2(1.0) | 16.0(1.1) | 15.5(0.7) | |
| | NONE | 13.3 (0.5) | 12.6 (0.5) | 15.7(0.7) | 15.8(0.5) | |
| | | | | | | |

### D. INSULIN AND GLUCOSE CONCENTRATION

Aliquots of K₃EDTA plasma were analysed to determine the concentrations of insulin (Ultrasensitive kit, DRG Diagnostics, Marburg/Lahn, Germany). Plasma glucose concentration was determined on an automated analyser (Cobas Mira Plus, Roche, Basle, Switzerland) using a colourimetric glucose oxidase-PAP kit (Randox, County Antrim, UK). Free fatty acids analyis were perfomed on EDTA plasma samples using the Wako 999-75406 NEFA-C kit and triglycerides analysis were performed on herparin plasma using a commercial kit.

Plasma insulin concentration generally decreased post-exercise but the overall temporal response was different depending on the trial (Figure 2). The plasma insulin concentration was significantly higher pre-exercise and 1h Post-Ex in the DC trial compared with the CON trial. Plasma insulin concentration was also higher pre-exercise in the CON trial compared with the fasting (NONE) trial. 1-way ANOVA on each trial independently showed no difference between the Rest and Pre-Ex values in the CON trial and a significant decrease below Pre-Ex levels Post-Ex and 1 h Post-Ex. Conversely, there was a significant increase above Rest at Pre-Ex in the DC trial and a significant decrease below Pre-Ex levels Post-Ex and 1 h Post-Ex. There was a significant decrease below Rest at Pre-Ex in the CON trial and a significant decrease below Pre-Ex levels Post-Ex and 1 h Post-Ex.

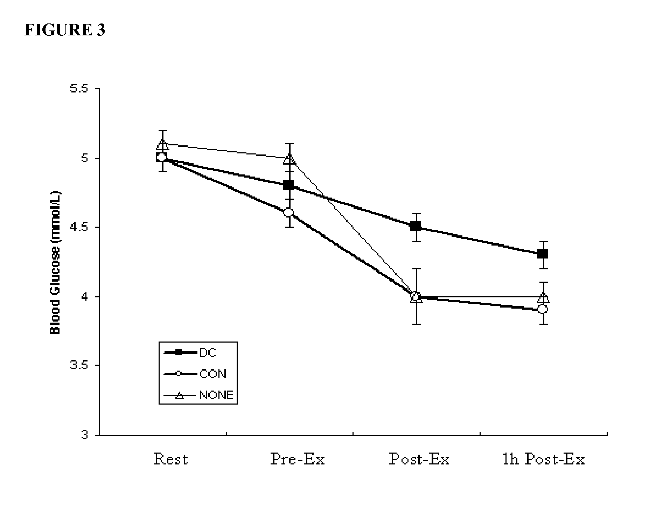

The plasma glucose concentration was significantly higher 1h Post-Ex in the DC compared with CON trial. 1-way ANOVA on each trial independently showed that there was a significant main effect of time for each trial (Figure 3). In the DC trial plasma glucose was not significantly different from Rest at Pre-Ex but was lower at Post-Ex and 1 h Post-Ex. It was not significantly lower than Pre-Ex at Post-Ex but was significantly lower at 1 h Post-Ex. In the CON trial plasma glucose was significantly lower than Rest at Pre-Ex, Post-Ex and 1 h Post-Ex. It was also significantly lower than Pre-Ex at Post-Ex and 1 h Post-Ex. In the NONE trial plasma glucose was not significantly different from Rest at Pre-Ex but was lower at Post-Ex and 1 h Post-Ex. It was also significantly lower than Pre-Ex at Post-Ex and 1 h Post-Ex. With the dark chocolate intervention (DC trial), the plasma glucose was significantly higher post exercise than with control (CON trial).

To summarise, dark chocolate (DC) consumption 2h before an acute bout of prolonged exercise (2.5 h at ∼ 60% VO₂max) may result in a significant insulinaemia and differential plasma glucose response compared with the consumption of control cocoa liquor-free chocolate (CON with a similar macronutrient and energy content) or NONE (fasting). Dark chocolate ingestion may also significantly elevate the plasma epicatechin concentration, slightly but significantly increase plasma antioxidant capacity and may also have beneficial effects on the plasma F₂-isoprostane concentration following exercise. The rating of perceived exertion may be lower, fatigue reduced, performance increased, and recovery may be faster after dark chocolate ingestion compared with control cocoa liquor-free chocolate.

## Claims

1. Use of at least 25mg of a polyphenol for the preparation of a product to be consumed before or during exercise for reducing fatigue, muscle soreness, perceived exertion or recovery time, and/or improving performance or cognition performance.

2. Use according to claim 1 wherein the exercise is prolonged and/or strenuous.

3. Use according to claim 1 wherein the product is consumed before the commencement of, or during, a bout of exercise.

4. Use according to claim 1 wherein the polyphenol is a cocoa polyphenol.

5. Use according to claim 1 wherein the polyphenol is epicatechin.

6. Use according to claim 1 wherein the polyphenol is catechin.

7. Use according to claim 1 wherein the polyphenol is an oligomer of epicatechin.

8. Use according to claim 1 wherein the amount of total polyphenol in the product (as measured by the folin assay in epicatechin equivalents) is from 50 mg to 2500 mg,

9. Use according to claim 5 wherein the amount of epicatechin in the product is from 30 mg to 200 mg.

10. Use according to claim 1 wherein the product containing the polyphenol to be consumed orally is cocoa liquor, cocoa powder, milk chocolate or dark chocolate, a cereal, a dairy product, an energy bar, a gel, a cocoa drink, a chocolate drink or a dietary supplement.

11. Use according to claim 10 wherein the dietary supplement is a capsule, tablet, pill, lozenge, dragee or powder.

12. Use according to claim 1 wherein the product is dark chocolate consumed before a bout of exercise in an amount from 30 to 150 gram.

13. Use according to claim 1 wherein the product contains up to 0.5 grams of caffeine and/or up to 1.0 grams of theobromine.

14. Use according to claim 1 wherein the product contains substantially no fat or sugar.

15. Use according to claim 1 also for
increasing fat mobilisation post-exercise, and/or
increasing beta-oxidation post-exercise, and/or
increasing gluconeogenesis post- and during exercise, and/or
increasing glycogenolysis post- and during exercise, and/or
decreasing overall body turnover of glucose post- and during exercise, and/or
decreasing post-exercise immunodepression, and/or
reducing post-exercise insulin changes, and/or
reducing post-exercise plasma free F2-isoprostane concentration, and/or
inhibiting the reduction of post-exercise plasma insulin (insulinaemia).

16. Use of at least 25mg of a polyphenol for the preparation of a product to be consumed before or during exercise for inhibiting a reduction in post-exercise blood glucose.

17. Use of at least 25mg of a polyphenol for the preparation of a product to be consumed before or during exercise for inhibiting a reduction in post-exercise blood insulin content.

18. Use of at least 25mg of a polyphenol for the preparation of a product to be consumed before or during exercise for increasing the post-exercise blood polyphenol content.

19. A method of reducing fatigue, muscle soreness, perceived exertion or recovery time, and/or improving performance or cognition performance by consuming a product comprising at least 25mg of a polyphenol before or during a bout of exercise..

20. A method of inhibiting a reduction in post-exercise blood glucose by consuming a product comprising at least 25mg of a polyphenol before or during a bout of exercise.

21. A method of inhibiting a reduction in post-exercise blood insulin by consuming a product comprising at least 25mg of a polyphenol before or during a bout of exercise.

22. A method of increasing the post-exercise blood polyphenol content by consuming a product comprising at least 25mg of a polyphenol before or during a bout of exercise.

23. Use of from 5 mg to 200 mg of a polyphenol for the preparation of a product to be consumed on a daily basis for reducing fatigue, muscle soreness, perceived exertion or recovery time, and/or improving performance or cognition performance.

24. A method of reducing fatigue, muscle soreness, perceived exertion or recovery time, and/or improving performance or cognition performance in a person taking regular exercise by consuming daily a product comprising from 5 to 200mg of a polyphenol.
